# EUROPÄISCHE PATENTSCHRIFT

(11) **EP 1 023 445 B1**
(45) Veröffentlichungstag und Bekanntmachung des Hinweises auf die Patenterteilung: **17.08.2005**
(21) Anmeldenummer: 98954421.8
(22) Anmeldetag: 15.10.1998
(51) Int. Cl.: C12N 15/12, C07K 14/475, C07K 16/22

(54) **CADHERIN DERIVED GROWTH FACTOR UND SEINE VERWENDUNG**
CADHERIN DERIVED GROWTH FACTOR AND THE APPLICATION THEREOF
FACTEUR DE CROISSANCE DERIVE DE LA CADHERINE ET SON UTILISATION

(30) Priorität: 15.10.1997 DE 19745284; 25.03.1998 DE 19813088
(43) Veröffentlichungstag der Anmeldung: 02.08.2000
(73) Patentinhaber: Pharis Biotec GmbH, 30625 Hannover (DE)
(72) Erfinder: FORSSMANN, Wolf-Georg, D-30625 Hannover (DE); STÄNDKER, Ludger, D-30625 Hannover (DE); MEYER, Markus, D-30625 Hannover (DE); MOSTAFAVI, Hossein, D-30163 Hannover (DE); OPITZ, Hans-Georg, D-69469 Weinheim (DE); KLING, Lothar, D-68167 Mannheim (DE)
(74) Vertreter: Meyers, Hans-Wilhelm, Dr.Dipl.-Chem.
(86) Internationale Anmeldenummer: PCT/EP1998/006547
(87) Internationale Veröffentlichungsnummer: WO 1999/019477

(56) Entgegenhaltungen:
- EP-A- 0 585 801
- US-A- 5 646 250
- HIDENOBU TANIHARA ET AL: "Cloning of five human cadherins clarifies characteristic features of cadherin extracellular domain and provides further evidence for two structurally different types of cadherin" CELL ADHESION AND COMMUNICATION, Bd. 2, 1. Januar 1994, Seiten 15-26, XP000576845
- SELIG S ET AL: "Molecular characterization of Br-cadherin, a developmentally regulated brain-specific cadherin" PROCEEDINGS OF THE NATIONAL ACADEMY OF SCIENCES OF USA., Bd. 94, Nr. 6, 18. März 1997, Seiten 2398-2403, XP002095155 WASHINGTON US

## Beschreibung

Gegenstand der vorliegenden Erfindung sind Peptide (Eiweißstoffe) mit zellproliferativen, zelldifferenzierenden und/oder zellprotektiven Eigenschaften, die als Cadherin Derived Growth Factor (CDGF) bezeichnet werden sowie ihre Verwendung.

Aufgabe der Erfindung ist es, Peptide bereitzustellen, die zellproliferativen, zellprotektiven und/oder zelldifferenzierenden Eigenschaften aufweisen.

Gelöst wird die Aufgabe durch als Cadherin Derived Growth Factor (CDGF) bezeichnete Peptide, deren Sequenz einer Teilsequenz eines Prä-Pro-Cadherins entspricht, wobei das Präpro-Cadherin die Domänen Signalsequenz, Pro-Sequenz, Cadherin repeats, Transmembranregion und intrazelluläre Domäne aufweist, dadurch gekennzeichnet, daß die Sequenz des Peptides die Pro-Sequenz umfaßt, mindestens eine der anderen Domänen des PräproCadherins fehlt und daß das Peptid zellproliferative, zellprotektive und/oder zelldifferenzierende Eigenschaften aufweist.

Cell Adhesion and Communication 2 (1994), 15 bis 26, offenbart die Klonierung von 5 humanen Cadherin und beschreibt extrazelluläre Domänen. Die zellproliferative Wirkung läßt sich beispielsweise an primären Osteoblasten aus Rattencalvarien, die zellprotektiven und/oder zelldifferenzierenden Wirkungen an primären Nervenzellkulturen aus Hinterwurzelganglien von Hühnerembryonen bestimmen.

In einer bevorzugten Ausführungsform ist der CDGF ein Fragment des Pro-Cadherins, d.h des um die Signalsequenz verkürzten Prä-Pro-Cadherins.

Besonders bevorzugt handelt es sich um den N-Terminus des Pro-Cadherins, also den Teil, der bei der Prozessierung des Pro-Cadherins zum Cadherin abgespalten wird, bzw. um ein N- oder C-terminal verkürztes Fragment davon.

Vorzugsweise weist der CDGF keinen Cadherin repeat auf.

Erfindungsgemäß bevorzugte Ausführungsformen sind Peptide mit der Sequenz Cadherin-1 human (28-154): Cadherin-2 human (24-159): Cadherin-3 human (27-107): Cadherin-4 human (21-169): Cadherin-5 human (26-47) : Cadherin-6 human (19-53): Cadherin-6 human (19-51): Cadherin-8 human: Cadherin-B human (Cadherin-11) Precursor (23-53): Cadherin-B human (Cadherin-11) Precursor (26-51): Cadherin-C human (Cadherin-12)-Brain-Cadherin Precursor (24-54): Cadherin-C human (Cadherin-12)-Brain-Cadherin Precursor (24-52): Cadherin-D human (Cadherin 13) (23-138): Cadherin-F human (Cadherin 14) (22-60):

Vorzugsweise ist das erfindungsgemäße Protein ein aus dem Menschen erhältliches Protein oder eine natürlich vorkommende humane Variante davon.

Ein Gegenstand der Erfindung ist auch ein neues Protein, das Teile der Aminosäuresequenz von CDGF umfaßt. Die Erfindung betrifft vorzugsweise einen CDGF, welcher die oben dargestellten Aminosäurensequenzen enthält, er kann jedoch auch Varianten dieser Sequenzen enthalten. Unter dem Begriff "Varianten" im Sinne der vorliegenden Erfindung sind Sequenzen zu verstehen, die durch Substitution, Deletion und/oder Insertion einzelner Aminosäuren oder kurzer Aminosäureabschnitte sich von den oben dargestellten Aminosäuresequenzen der CDGF unterscheiden.

Unter dem Begriff "Varianten" fallen sowohl natürlich vorkommende allelische Variationen der CDGF sowie durch rekombinante DNA-Technologie (insbesondere durch in vitro-Mutagenese mit Hilfe von chemisch synthetisierten Oligonukleotiden) erzeugte Proteine, die hinsichtlich ihrer biologischen und/oder immunologischen Aktivität den CDGF entsprechen.

Konservative Austausche sind beispielsweise Y für/gegen V, K für/gegen S, A für/gegen S, D für/gegen E, G für/gegen S, R für/gegen Q, R für/gegen A, Q für/gegen K.

Erfindungsgemäß beansprucht werden auch Nukleinsäuren, die für die Peptide oder Derivate codieren oder komplementär zu diesen Nukleinsäuren sind. Bei den Nukleinsäuren kann es sich beispielsweise um DNA, RNA, PNA oder nuclease-resistente Analoga handeln. Die erfindungsgemäßen Nukleinsäuren eignen sich zur Expression der CDGF in vivo im Sinne einer Gentherapie sowie als Antisensenukleotide zur Verringerung der Expression. Auch Vektoren, die die Nukleinsäuren enthalten sind Gegenstand der Erfindung. Die Vektoren eignen sich insbesondere zur Expression des Peptides in gentechnisch veränderten Organismen.

Desweiteren betrifft die Erfindung Antikörper, die gegen CDGF oder Derivate davon gerichtet sind sowie Antagonisten/Inhibitoren, die gegen CDGF, ein Derivat oder eine der erfindungsgemäßen Nukleinsäuren gerichtet sind. Diese Substanzen eignen sich als Arzneimittel zur Behandlung von Zuständen, die mit einer Überexpression der CDGF verbunden sind sowie zum Einsatz in der Diagnostik.

Die erfindungsgemäßen CDGF, Derivate, Verbindungen, Nukleinsäuren, Antikörper und/ oder Antagonisten/Inhibitoren können zusammen mit üblichen Hilfsmitteln als Arzneimittel verwendet werden. Es wird besonders bevorzugt, daß die Arzneimittel in geeigneten galenischen Zubereitungen zur oralen, bukalen, intravenösen, intramuskulären, intracutanen, intrathekalen, intranasalen, lokal-topischen Anwendung sowie als Aerosol zur transpulmonalen Applikation vorliegen.

Die Menge an zu verabreichendem Arzneimittel beträgt bevorzugt 1 µg bis 1 g pro Darreichungseinheit pro Tag.

Die erfindungsgemäßen Arzneimittel eignen sich zur Behandlung und Prophylaxe von degenerativen sowie metabolischen Erkrankungen des Knochens wie Osteoporose, Osteomalazie und Osteopenie, des Pankreas wie Diabetes mellitus, des Muskels wie Muskeldystrophien, der Gefäße, des zentralen und peripheren Nervensystems wie periphere und zentrale Neuropathien, der Lunge wie Asthma bronchiale, des Magens wie Ulcus, sowie zur Therapie und Prophylaxe von entzündlichen Prozessen, gestörten Entzündungsreaktionen, Tumorerkrankungen, sowie zur Wund- und Knochenheilung.

Das erfindungsgemäße Diagnostikmittel enthält poly- oder monoklonale Antikörper gegen das erfindungsgemäße Peptid, gegebenenfalls in markierter Form, wie in fluoreszenz- oder radioaktiv-markierter Form, um in einem an sich bekannten ELISA oder RIA eingesetzt zu werden. Das erfindungsgemäße Diagnostikmittel enthält DNA, RNA und/oder PNA gegebenenfalls in modifizierter und/oder markierter Form zum Einsatz in dem Fachmann bekannten Testsystemen wie PCR oder Fingerprinting.

Die Diagnostikmittel eignen sich zur Kontrolle von CDGF-Spiegeln in Geweben, in Sekreten und/oder in Körperflüssigkeiten wie Plasma, Urin und Liquor cerebrospinalis sowie als Marker für Funktionsstörungen des Knochens, der Muskeln, der Gefäße, des Nervensystems, der Lymphorgane, des Magen-Darmtraktes, des Immunsystems und von Diabetes sowie inflammatorischen und neoplastischen Prozessen sowie als Marker bei Krebs (Tumormarker).

Die erfindungsgemäßen CDGF und ihre Derivate sind durch Isolierung erhältlich aus Hämofiltrat durch Kationenaustauscher-Extraktion mit nachfolgender Elution der adsorbierten Substanzen, eine erneute Kationenaustauscher-Chromatographie des die Peptide enthaltenden Extraktes sowie mehrstufige Umkehrphasen-Chromatographie. Totalsynthetisch sind die erfindungsgemäßen Peptide durch Festphasensynthese im Sinne der Merrifield-Synthese oder Flüssigphasensynthese nach dem Fachmann an sich bekannten Methoden mit geschutzten Aminosäuren und anschließende Aufreinigung erhältlich. Auch durch dem Fachmann an sich bekannte Verfahren der heterologen Expression mittels gängiger biotechnologischer Vektoren lassen sich die erfindungsgemäßen Peptide herstellen.

Beispielsweise wurde ein Peptid, das als OB-CDGF bezeichnet wird, mit chromatographischen Methoden aus humanem Hämofiltrat aufgereinigt und mit Hilfe eines Bioassays identifiziert.

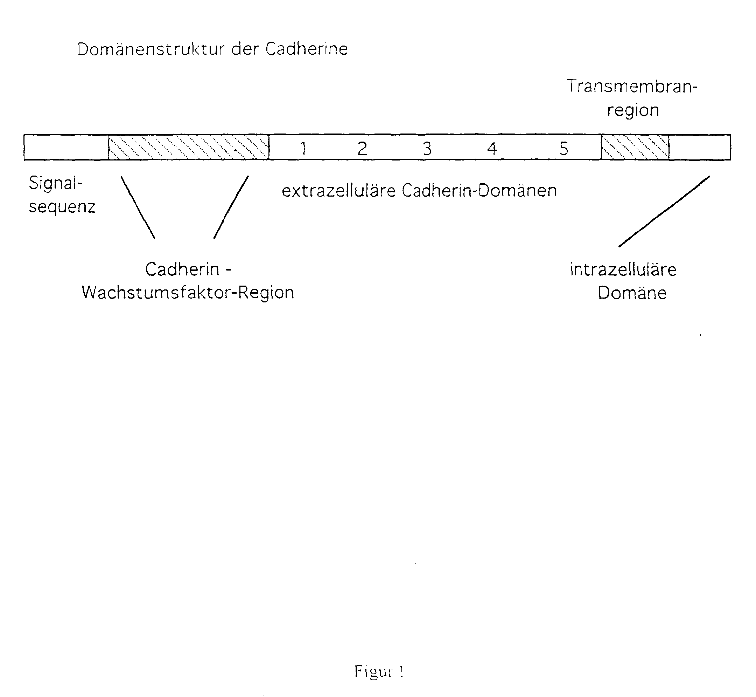

Das Peptid besitzt eine Molekularmasse von 3062.8 Da. Bislang wurde über die Analyse einer cDNA eine OB-Cadherin Prä-Pro-Sequenz postuliert. In dieser aus der cDNA abgeleiteten Sequenz findet sich die erfindungsgemäße Peptidsequenz direkt hinter der putativen Signalsequenz (siehe Figur 1).

Die biochemische Charakterisierung des erfindungsgemäßen Peptides erfolgte durch Massenspektrometrie und Sequenzierung des gesamten Peptides. Die Sequenzanalyse des biologisch aktiven Peptides ergab die folgende Aminosäuresequenz für OB-CDGF:

Im ESI (Elektrospray Ionisation)-Massenspektrum des OB-CDGF wurde die Molekularmasse (MW) bestimmt mit:
OB-CDGF, MW 3062.8 Da

Das erfindungsgemäße Peptid ist durch ein Reinigungsverfahren ausgehend von humanem Hämofiltrat erhältlich. Dieses Verfahren gemäß DE 36 33 707, welches die Gewinnung von Eiweißstoffen aus Hämofiltrat offenbart, wurde in einer modifizierten Form durchgeführt.

Hämofiltrat fällt bei der Ultrafiltration des Blutes von Nierenkranken in großen Mengen an. Das humane Hämofiltrat wird gegebenenfalls mit Wasser verdünnt und angesäuert. Der pH-Wert beträgt vorzugsweise 1.5 bis 3.5, insbesondere 2.5 bis 3.0. Danach wird das Hämofiltrat über einen Kationenaustauscher geleitet, beispielsweise einem mit Sulfonsäuregruppen modifizierenden Trägermaterial (Fraktogel SP - 650 (M), Merck, Darmstadt). Die an den Kationenaustauscher gebundenen Peptide werden mit einer relativ hoch konzentrierten Salzlösung eluiert. Die Ionenstärke der Elutionslösung entspricht dabei ungefähr einer 0.5 bis 1 molaren Ammoniumacetatlösung.

Das aufgefangene Eluat wird einer weiteren Kationenaustauscher-Chromatographie unterzogen. Diese Chromatographie ist vorzugsweise eine Stufenelution mit Puffern von ansteigenden pH-Werten.

Die das erfindungsgemäße Peptid enthaltenen Fraktionen werden mittels präparativer Umkehrphasen-Chromatographie und nachfolgender semipräparativer Umkehrphasen-Chromatographie beispielsweise an C4-modifizierten Trägermaterialien weitergereinigt. Der Reinigungsgrad wird vorzugsweise mittels analytischer Umkehrphasen-Chromatographie an C18-modifizierten Trägermaterialien überprüft.

Die durch die chromatographische Reinigung erhaltene Substanz wurde der Strukturaufklärung zugeführt. Die Bestimmung der Molekülmasse des nativen Peptids erfolgte mittels eines Elektrospray-Massenspektrometers. Die Sequenzanalyse erfolgte über einen Edman-Abbau der Peptide sowie von chemisch modifizierten Derivaten mit einem ABI 473 A Sequenzer.

Die Totalsynthese erfolgte an üblichen Festphasen im Sinne der Merrifield-Synthese. Die Synthesestrategie und der Aufbau des Peptids und von ihm abgeleiteten Derivaten mit den entsprechend geschützten Aminosäuren sind dem Fachmann bekannt.

Das erfindungsgemäße OB-CDGF sowie seine cDNA, sein Gen und Analoga, Fragmente und Derivate zu dem Peptid, der cDNA und dem Gen sowie Antikörper, welche die Wirkung des OB-CDGF aufheben, können als Arzneimittel Verwendung finden.

Figur 1 zeigt die Domänenstruktur der Cadherine.

Figur 2 zeigt die Chromatogramme der Isolierung des OB-CDGF aus Hämofiltrat. Die Identifizierung erfolgt durch ein Proliferationsassay an primären Osteoblasten. Einzelheiten der Aufreinigung können dem Beispiel 1 entnommen werden.

Figur 3 zeigt das Elektrospray-Massenspektrum des isolierten OB-CDGF. Aus den zweifach und dreifach protonierten Ionen berechnet sich ein Molekulargewicht vom 3.062 Dalton.

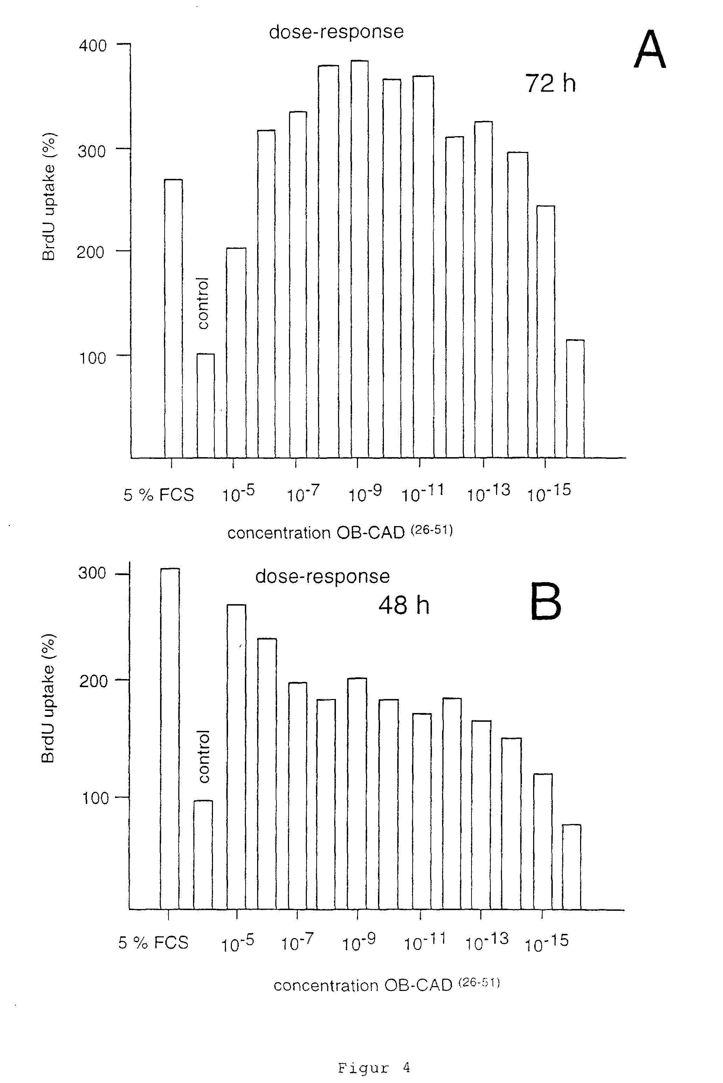

Figur 4 zeigt die Dosis-Wirkungs-Kurve von chemisch synthetisiertem OB-CDGF auf die Proliferatin von primären Knochenzellen. In Figur 4a wird die Wirkung für eine Inkubationszeit von 72 Stunden, in Figur 4b für eine Inkubationszeit von 48 Stunden gezeigt. Gemessen wurde die Aufnahme von Bromdesoxyuridin (BrdU). Der Einbau von BrdU wurde mittels ELISA gemessen.

Figur 5 zeigt die spezifische Bindung von iodmarkiertem OB-CDGF an primäre Osteoblasten (monolayer) und an die Extracellular Matrix (ecm) von primären Osteoblasten. Dargestellt ist die Verdrängung des markierten OB-CDGF durch nichtmarkiertes OB-CDGF bei steigender Konzentration der Menge an nichtmarkiertem OB-CDGF.

Figur 6 zeigt die Stimulation von primären Osteoblasten durch OB-CDGF Zugabe. Die intracelluläre Calciumkonzentration steigt durch Zugabe von OB-CDGF an. Gemessen wurde der Calciumgehalt in Gegenwart von Fura 2 an Einzelzellen im Fluoreszenzmikroskop.

Figur 7 ist ein Westernblot, der die intracelluläre MAP-Kinaseaktivität nachweist. Diese Aktivität gilt als Parameter für die Rezeptor- und Calcium-vermittelte Zellantwort auf OB-CDGF an primären Osteoblasten. Die obere Bande (46 kD) zeigt die Konzentration der MAP-Kinase nach Stimulation mit fötalem Kälberserum FCS (links) oder OB-CDGF (rechts) in Abhängigkeit der Inkubationszeit. Die untere Bande zeigt eine interne Kontrolle für den Zellaufschluß/Westernblot.

Die Erfindung wird durch die folgenden Beispiele erläutert.

### Beispiel 1

### Hämofiltrat-Batch-Extraktion

800 bis 1.000 L Hämofiltrat werden mit HCl auf einen pH -Wert von 2.7 eingestellt und mit Wasser auf eine Leitfähigkeit von 5.5 mS/cm verdünnt und mit einer Flußrate von 3 L/min auf einen starken Kationenaustauscher aufgetragen.

### Chromatographiebedingungen:

- Säule:: Vantage VA 250 (Amicon, Witten)
- Säulenmaterial:: Fractogel TSK SP 650 (M), 25 cm x 20 cm
- Fluß:: 3 L/min
- Detektion:: 280 nm, pH, Leitfähigkeit
- Puffer A:: Hämofiltrat pH 2.7, Leitfähigkeit 5.5 mS/cm
- Puffer B:: 0.5 M Ammoniumacetat
- Anlage:: Autopilot Chromatographiesystem, (PerSeptive Biosystems, Wiesbaden)

Nach Auftrag der insgesamt 1.000 L Flüssigkeit über Nacht wird mit mehreren Säulenvolumina 5 mM HCl gespült. Die Elution der gebundenen Peptide erfolgt als Batch-Elution mit 0.5 M Ammoniumacetat. Hierbei wird eine komplette Elution der Peptide über steigenden pH-Wert (6.8 - 7.2) und steigende Leitfähigkeit (56 mS/cm) in etwa 5 L Eluat erreicht.

### Erste präparative Auftrennung

Die Ammoniumacetat-Eluate der Batch-Extraktion werden in Mengen von 5.000 bis 10.000 L Hämofiltrat-Peptid vereinigt. Nach pH-Einstellung auf pH 2.7 wird der Peptidextrakt unter Zumischung von VE-Wasser mit einer Leitfähigkeit von 5.5 mS/cm auf den präparativen Kationenaustauscher aufgetragen.

### Chromatographiebedingungen:

- Säule:: Vantage 250 VA
- Säulenmaterial:: Fractogel TSK SP 650 (M), 25 cm x 20 cm
- Fluß:: bis zu 3 L/min während des Auftrages 0.5 bis 1 L während der Elution
- Detektion:: 280 nm, pH, Leitfähigkeit
- Probe:: Hämofiltrat pH 2.7, Leitfähigkeit 5.5 mS/cm
- Anlage:: Autopilot Chromatographiesystem, (PerSeptive Biosystems, Wiesbaden)

Nach Auftrag des Rohextraktes über 240 min wird die Säule mit 0.01 M HCl gespült, bis die Leitfähigkeit unter 1 mS/cm ist. Die Elution erfolgt dabei in mehreren Stufen mit den im folgenden angegebenen Puffern

| Puffer (mS/cm) | pH-Wert | Puffersubstanzen | Leitfähigkeit |
|---|---|---|---|
| Waschpuffer | 2.0 | 0.01 M HCl | 1 |
| Elutionspuffer 1 | 3.6 | 0.1 M Zitronensäure-1-hydrat | 2.9 |
| Elutionspuffer 2 | 4.5 | 0.1 M Essigsäure + 0.1 M Natriumacetat | 4.0 |
| Elutionspuffer 3 | 5.0 | 0.1 M Äpfelsäure | 6.2 |
| Elutionspuffer 4 | 5.6 | 0.1 M Bernsteinsaure | 6.1 |
| Elutionspuffer 5 | 6.6 | 0.1 M NaH₂PO₄ | 4.9 |
| Elutionspuffer 6 | 7.4 | 0.1 M NaH₂PO₄ | 6.7 |
| Elutionspuffer 7 | 9.0 | 0.1 M Ammoniumcarbonat | 6.7 |

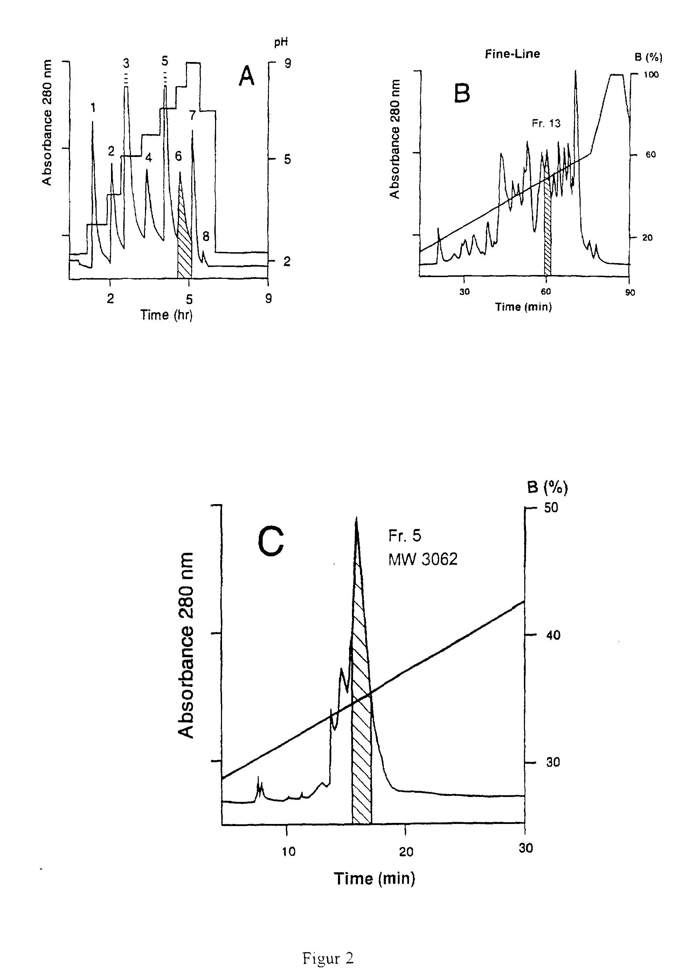

Die Eluate 1-7 werden als pH-Pool I-VII bezeichnet. Sie werden separat gesammelt. Die Elution erfolgt bis zum Erreichen einer neuen Basislinie, wobei für die einzelnen pH-Pools I bis VII Elutionsvolumina von 10 bis 25 L erreicht werden. Der Chromatogramm ist in Figur 2a gezeigt.

### Zweite präparative Auftrennung:

Die einzelnen pH-Pools werden zur Fraktionierung und gleichzeitigen Entsalzung über eine Reversed Phase Chromatographie getrennt

### Chromatographiebedingungen:

- Säule:: FineLine 100 (Pharmacia, Freiburg)
- Säulenmaterial:: Source RPC, 15 Fm 10 x 12.5 cm (FineLine 100)
- Fluß:: 150 mL/min (FineLine 100)
- Detektion:: 280 nm, Leitfähigkeit, pH
- Puffer A:: 10 mM HCl
- Puffer B:: 80% Acetonitril in 10 mM HCl
- Gradient:: 0-60% Puffer B in 5 Säulenvolumen

Nach Auftrag der pH-Pools wird die Säule mit Puffer A gespült. Während der Elution werden Fraktionen zu 200 ml gesammelt. Das Chromatogramm ist in Figur 2 b gezeigt. Aliquots der Fraktionen werden im Bioassay getestet. Die Fraktionen werden gefriergetrocknet und bei -20EC gelagert.

### Semipräparative Reverse-Phase C18-Chromatographie:

Die im Bioassay aktive Fraktion 13 aus pH-Pool VI wurde über eine semipräparative Reverse-Phase Säule aufgetrennt. Die Fraktionen 5-7 enthielten die erfindungsgemäße Substanz.

### Chromatographiebedingungen:

- Säule:: 4,7 cm x 30 cm Stahlsäule
- Füllmaterial:: Vydac RP-C18 15-20 Fm, 300 Å
- Puffer A:: 0,1 % TFA
- Puffer B:: 0,1 % TFA, 80 % Acetonitril
- Gradient:: 5 - 50% B in 45 min, 50 - 100% B in 10 min
- Fluß:: 42 ml/min
- Detektion:: 214 nm und 280 nm
- Chromatographieanlage:: BioCad
- Fraktionen:: ä 1.5 min ab Start des Gradienten

Das Chromatogramm ist in Figur 2c gezeigt.

### Massenbestimmungen

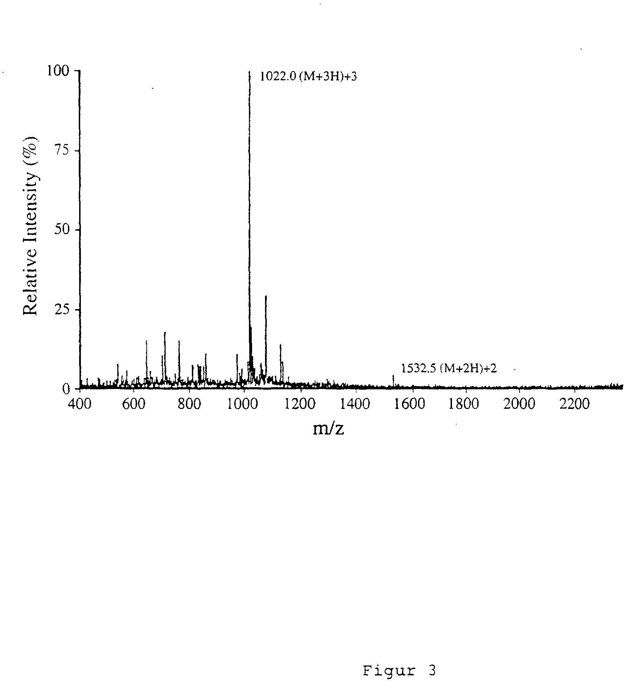

Alle Massenbestimmungen der nativen und synthetischen Peptide wurden auf einem Elektrospray-Massenspektrometer (ESI-MS) durchgeführt. Die Molekülmassen des Peptids wurden entsprechend der oben gezeigten Massenzahlen (MW) bestimmt. Das Massenspektrum ist in Figur 3 gezeigt.

### Sequenzbestimmung

Das gereinigte, native und das synthetisch hergestellte Peptid wurde mittels Edman-Abbau auf einem ABI 473 A Sequenzer unter Verwendung des Standard-Programms analysiert. Die Proben werden auf eine Polybrene-Membran in Mengen zwischen 100 und 400 pmol aufgetragen. In Übereinstimmung mit den Ergebnissen der Massenbestimmungen ergab sich folgende Aminosäuresequenz:

### Datenbankvergleich

Ein Datenbankvergleich wurde mit Hilfe des HUSAR-Programmpakets an den SwissProt und EMBL-Nukleinsäure Datenbanken durchgeführt. Die Sequenz entspricht den aus der cDNA abgeleiteten Aminosäuren des humanen Cadherin-11 Precursors (Osteoblaten Cadherin, Propeptid, Aminosäuren 26-51).

### Resynthese

Die Synthese des Peptids mit der Sequenz ERRGHLRPSFHGHHEKGKEGQVLQRS wurde nach der Merrifield-Festphasen-Methode ausgehend von geschützten Fmoc-Aminosäuren durchgeführt. Das synthetische Peptid wurde über Umkehrphasen-Chromatographie aufgereinigt. Die Identität und Reinheit der Substanz wurde mit Hilfe von Massenspektrometrie, Sequenzanalyse und Kapillarzonenelektrophorese nachgewiesen.

### Bestimmung der biologischen Aktivität von OB-CDGF (zellproliferativer Effekt)

Die Isolierung des OB-CDGF erfolgte anhand seiner biologischen Aktivität in einem Proliferationssassay der primären Osteoblasten. Dazu wurden jeweils Aliquots der unter Beispiel 1 beschriebenen einzelnen Chromatographiestufen gefriergetrocknet und anschließend dem biologischen Assay zugeführt. Die Fraktionen, die jeweils ein positives Signal ergaben, wurden der weiteren Reinigung unterzogen.

Der Assay mißt die Proliferation der Zellen, nachdem sie in serumfreien Medium mit 1 mg/ml Rinderserumalbumin gehalten wurden, dann die Proben zugegeben wurden und nach weiteren 48 Stunden der Einbau von ₃H-Thymidin oder Bromdeoxyuranosin (Brdu) bestimmt wurde. Als Positiv-Kontrolle wird in diesem Assay knochenwachstumsfördernde Faktoren wie IGF oder Angiotensin oder fötales Kälberserum (FCS) eingesetzt.

Die Experimente wurden in Anlehnung an Pfeilschifter et al., Endocrinology 126, 703, 1990, durchgeführt.

Die Gewinnung von primären Osteoblasten erfolgte durch sequentielle Abdauung mittels Collagenase aus fetalen Rattenkalvarien. Dabei wurden 5 Zellfraktionen erhalten. Der Pool aus den Zellfraktionen 3-5 wurde in vitro kultiviert. Die Kultur der Zellen erfolgte in einem Inkubator bei einer relativen Luftfeuchte von 95%, einem CO₂-Gehalt von 5% und einer Temperatur von 37EC. Die Untersuchungen der Prüfsubstanzen erfolgte in Kulturen der ersten, zweiten oder dritten Zellpassage.

Für die Untersuchung wurden die Zellen mindestens 95 Stunden vor Aufgabe der Prüfsubstanzen in einer Zellzahl von 7x10³ Zellen (in 100 µl Kulturmedium) / Well in Mikrotiterplatten (MTP) mit Rundboden ausgesät. Dabei fand als Kulturmedium MEM Dulbecco (plus 4,5 g/l Glukose plus 3,7 g/l NaHCO₃ ohne Glutamin) Verwendung, dem 5% fetales Kälberserum (FKS) und 5000 U/ml Penicillin/Streptomycin zugesetzt werden.

Unmittelbar vor Zugabe der Prüfsubstanzen zur Zellkultur erfolgte ein Austausch des Mediums gegen 150 µl Medium, das anstelle von FKS 1 mg/ml Rinderserumalbumin (RSA) enthielt. Prüfsubstanzen wurden in den gewünschten Konzentrationen dem RSA-haltigen Medium zugesetzt. Als Positivkontrolle wurde TGFβ₁ (Transforming growth factor β₁) in Konzentrationen von 0,1-0,2 ng/ml mitgeführt. Pro (Positiv-) Kontrolle bzw. Substanzkonzentration wurden Dreifachbestimmungen durchgeführt.

Die Inkubation der Zellkulturen mit Prüfsubstanzen erfolgte 24 bis 72 Stunden, in den letzten 3 Stunden zusätzlich unter Anwesenheit der Thymidinsonde (Zugabe von 1 µCi Methyl-³H-Thymidin/MTP-Vertiefung in 20 µl PBS-Lösung).

Am Ende der Inkubationszeit wurden die Zellkulturen 3x mit 0,9%iger Kochsalzlösung gewaschen und anschließend mit je 100 µl Flüssigszintillator (OptiPhase Supermix^{J} der Firma Wallac) versetzt. Im Anschluß daran erfolgte die Vermessung der in die DNA eingebauten Radioaktivität in einem Flüssigszintillationscounter (1450 MicroBeta^{J} der Firma Wallac) in cpm.

Bei der Auswertung dienen Zellkulturen, die ausschließlich RSA-haltiges Medium erhalten hatten, als Kontrollen (100%).

Der OB-CDGF besitzt in dosisabhängiger Weise eine wachstumsfördernde Wirkung auf primäre Osteoblasten (Knochenzellen). Diese biologische Wirkung wurde sowohl für das native als auch für das synthetisch hergestellte Peptid nachgewiesen.

### Beispiel 2

### Bestimmung der biologischen Aktivität BR-CDGF

Das der CDGF-Region des Cadherin-12 (BR-Cadherin; N-Cadherin-2) entsprechende Peptid mit der Aminosäuresequenz QPQPQQTLATEPRENVIHLPGQRSHFQRV, welches nach der Festphasensynthese - wie unter Beispiel 1 für das OB-CDGF beschrieben - synthetisiert wurde, wurde auf seine überlebensfördernde Wirkung auf Primärkulturen von Neuronen aus Hinterwurzelganglien von Hühnerembryonen getestet.

### Das Testmodell ist folgendermaßen aufgebaut:

### Primäre Nervenzellkulturen aus Hinterwurzelganglien von Hühnerembryonen (Embryonalentwicklung E10)

Die Präparation und Kultivierung erfolgt wie beschrieben von Borasio G.D., John J., Wittinghofer A., Barde Y.-A., Sendtner M. Heumann R. (1989) Neuron 2, 1087-1096. Zur Bestimmung von uberlebensfördernden Effekten von Wirkstoffen wird die Bestimmung des zellulären LDH-Gehalts nach einer Inkubationszeit von 48 Stunden eingesetzt. Hierzu wird das Kulturmedium durch Ausklopfen der Platte entfernt. Nur die vitalen Zellen bleiben am Plattenboden haften und können dann bestimmt werden. Zur Bestimmung des LDH-Gehalts wird der LDH-Zytotoxizitätstestkit von Boehringer Mannheim (BM) eingesetzt.

Die Kulturen werden in 96-Well Mikrotiterplatten als Duplikate eingesetzt. Auf jeder Platte wird eine NGF-Eichkurve mitgeführt und die biologische Aktivität der Substanzen in pg/mL NGF-Äquivalenten ausgedrückt. Als niedermolekulare Referenzsubstanz wird das Staurosporin-Derivat K252a in einer Konzentration von 300 nmol/L mitgeführt.

Das Peptid zeigte in konzentrationsabhängiger Weise eine überlebensfördernde Wirkung auf diese primären Neuronen. Diese Zellen sind typische Nervenzellen, so daß BR-CDGF ein neuroprotektiver Faktor ist.

### Beispiel 3

### Bestimmung der biologischen Aktivität CAD6-CDGF (zellprotektiver Effekt)

Das der CDGF-Region des Cadherin-6 entsprechende Peptid mit der Aminosäuresequenz TLSTPLSKRTSGFPAKKRALELSGNSKNELNRS, welches nach der Festphasensynthese - wie unter Beispiel 1 für das OB-CDGF beschrieben - synthetisiert wurde, wurde auf seine überlebensfördernde Wirkung auf Primärkulturen von Neuronen aus Hinterwurzelganglien von Hühnerembryonen getestet. Das Testmodell ist, wie in Beispiel 2 beschrieben, aufgebaut.

Das Peptid zeigte in konzentrationsabhängiger Weise eine überlebensfördernde Wirkung auf diese primären Neuronen.

### Beispiel 4

### Bindungsassay von markiertem OB-CDGF an Osteoblasten

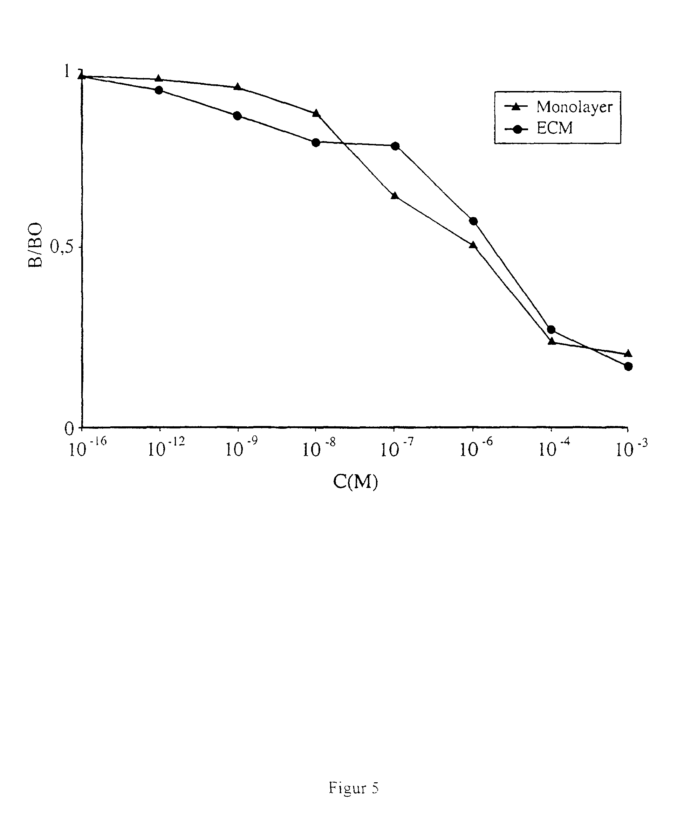

Die Bindung der osteoproliferativen Peptide an neonatale Rattenosteoblasten Monoschichten wurde unter Verwendung von ¹²⁵I markierten Peptiden durchgeführt. Die Iodierung erfolgte durch Chloramin T. Die spezifische Aktivität der Peptide betrugt 100.000 cpm/ng. Die Bindungsstudien wurden durchgeführt an confluenten Schichten sowie der Extracelluar-Matrix einer ersten Passage von neonatalen Rattenosteoblasten. Die Zellen wurden serumhaltigem Medium für 24 Stunden inkubiert und dann vier mal mit PBS gewaschen und in 250 µl des Assaypuffers (20 mM HEPES, 0,1 mg/ml BSA, pH 7,0) mit 80.000 cpm des iodmarkierten Peptids in Gegenwart unterschiedlicher Konzentrationen des unmarkierten Peptids für 2 Stunden bei 4°C inkubiert. Dann wurde der Inkubationspuffer entfernt und die Zellen viermal mit kaltem PBS gewaschen und mit 1 M NaOH solubilisiert. Die zellgebundene Aktivität wurde mit Hilfe eines Gamma-Counters bestimmt. Die entsprechenden Versuchsergebnisse sind in Figur 5 dargestellt. Mit Zunahme der Konzentration an nichtmarkiertem OB-CDGF nimmt die Menge an spezifisch gebundenem markierten OB-CDGF ab.

### Beispiel 5

### Intracelluläre Calciumfreisetzungen in primären Osteoblasten

Die intracellulare Signalkette für Calcium²⁺ wird an einzelne Osteoblastenzellen gemessen. Die intracelluläre Ca²⁺-Aktivität von primären Osteoblasten wird mit dem Ca²⁺ empfindlichen Farbstoff Fura-2 (Calbiochem, Bad Soden, Deutschland) gemessen. Die primären Osteoblasten werden auf Glasträgern (Durchmesser 30 mm) für ein bis drei Tage kultiviert, die an einer Perfusionskammer eines invertierten Mikroskops (Axiomat IDC-UV, Zeiss, Göttingen, Deutschland) befestigt ist. Die Zellen wurden in einer modifzierten Krebs-Henseleit-Pufferlösung (KHB, Zusammensetzung 145 mM NaCl, 1,6 mM K₂HPO4, 0,4 mM KH₂PO₄, 1,3 mM Calciumgluconat, 1,0 mM MgCl₂, 5,0 mM D-Glucose, pH 7,4) für 10 min bei 37°C mit 5 µM Fura-2/Acetoxymethylester gelöst in 0,1 g/l Pluronic F-127 (Sigma, Deisenhofen, Deutschland) in RPMI 1640 (PAA, Cölbe, Deutschland) inkubiert.

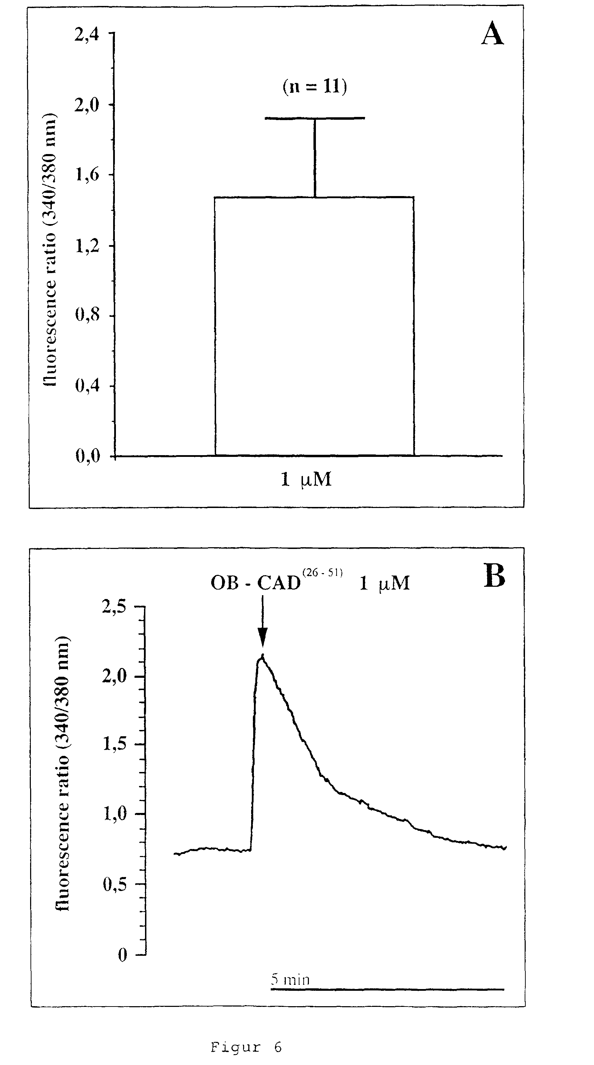

Der Inkubation folgt eine Äquilibrierungsperiode von 10 bis 15 min in der die Zellen mit KHB-Lösung bei 37° mit einer Perfusionsrate von etwa 5 ml/min gespült werden. Die Fura-2- beladenen primären Osteoblasten werden bei 340, 360, 380 nm mit einem Hochgeschwindigkeitsfilterrad (Rotationsgeschwindigkeit 10 Herz), einem Einzelphotonzählrohr (Hamamatsu, Herrsching, Deutschland) und einer Xenonquarzlampe (XBO 75 W/OFR, Zeiss, Deutschland) als Licht-/Impulsquelle gemessen. Die Fu-ra-2-Emission wird durch ein Langwellenfilter bei 510 nm mit einer Ultrafluar 125 x Glycerolimmersionslinse gemessen. Alle Messungen werden an einzelnen Zellen durchgeführt. Das Meßfeld wird mittels eines justierbaren Loch mit etwa 8 µm Durchmesser gewählt. Ausgewertet wird das Verhältnis der Photonenemission nach Anregen bei 340 und 380 nm. Die Rohdaten werden auf Autofluoreszenz und Rauschen korrigiert. Der so erhaltene Hintergrundwert wird von den Meßsignalen bei jedem Experiment abgezogen. Die Rohdaten werden berechnet und 10 benachbarte Datenpunkte werden gemittelt, um eine Zeitauflösung von 1 Hz zu erhalten. Die Fluoreszenz bei 360 nm wird verwendet um die Calciumaktivität zu prüfen. Alle Proben werden vor dem Experiment in einem Phosphatpuffer gelöst und wenigstens für 1 bis 2 Minuten gemessen. Die Kalibrierung des Ca²⁺ wird am Ende jedes Experiments durch Inkubation der Zellen mit dem Ca²⁺-Ionophore, Ionomycin (10 µmol, Sigma, Deisenhofen, Deutschland) in Gegenwart von 1,3 mmol Ca²⁺ bzw. nominaler Abwesenheit (5 mM Ethylenglycol-bis(β-aminoethyl ether), N,N,N',N'-Tetraessigsäure, in Gegenwart von EGTA) gemessen. Nur Kalibrierungen sowohl mit Maximum- als auch Minimumwerten werden für die Bestimmung des Ca²⁺-Wertes verwendet. Bei den Experimenten, bei denen die doppelte Kalibrierung nicht erfolgreich war, wird der Mittelwert aller Kalibrierung verwendet. Die entsprechenden Ergebnisse sind in Figur 6 gezeigt.

### Beispiel 6

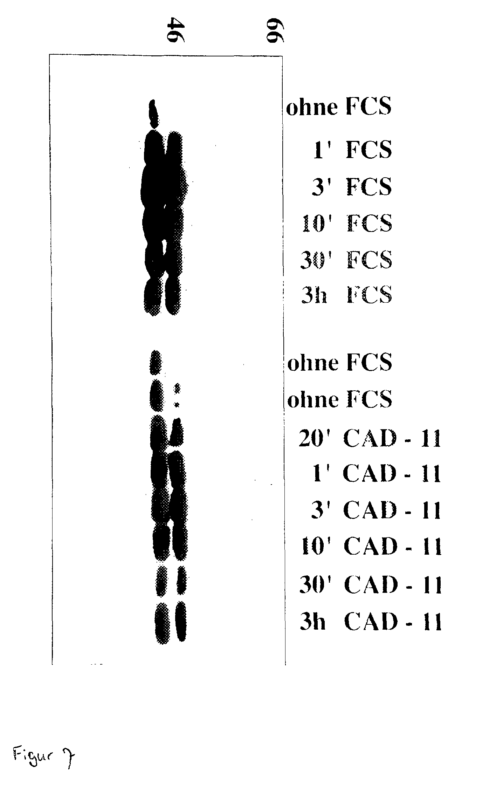

Wie bereits unter Beispiel 5 gezeigt, kann durch die Inkubation mit CDGF eine intrazelluläre Calcium-Freisetzung an primären Osteoblasten induziert werden. Um diese physiologische Antwort näher zu charakterisieren wurde der weitere intrazelluläre Signalweg ("down stream" vom Calcium) untersucht. Eine mögliche Signaltransduktionskaskade ist die Aktivierung der MAP-Kinase, ein Enzym, das eine wesentliche Rolle bei der Signalvermittlung vom Zytoplasma in den Zellkern spielt. Bei der Weiterleitung des Signals kommt es zu einer Phosphorylierung des Enzyms, das mit einem spezifischen Antikörper detektiert werden kann. Der Nachweis dieses Proteins kann somit im Westernblot erfolgen und stellt ein Maß für die Phosphorylierung wie auch für die Expression aufgrund des Stimulus dar. Primäre Osteoblasten wurden über verschiedene Zeiten mit Cadherin bzw. Kontrollen (FCS) inkubiert. Durch Zugabe des Antikörpers wurde im Westernblot die Expression des Enzyms (MAP-Kinase) in der Zelle nachgewiesen. Die Ergebnisse sind in Figur 7 dargestellt. Es ist deutlich sichtbar, daß ohne einen Stimulus wie FCS oder OB-CDGF keine Expression stattfindet, während nach Stimulation eine deutliche Bande in der erwarteten Größe der MAP-Kinase erscheint (obere Bande). Je länger mit OB-CDGF stimuliert wurde, desto deutlicher wurde die erkennbare Bande. Dies zeigt eindeutig, daß OB-CDGF zu einer Expression der intrazellulären MAP-Kinase führt. Hieraus läßt sich schließen, daß CDGF rezeptorvermittelt über Calcium wirkt und die weitere Signalverarbeitung über die MAP-Kinase verläuft.

## Patentansprüche

1. Als Cadherin Derived Growth Factor (CDGF) bezeichnetes Peptid, **dadurch gekennzeichnet, dass** es sich um ein Peptid handelt, das bei der Prozessierung von Pro-Cadherin zu Cadherin als Pro-Sequenz abgespalten wird oder um ein Fragment davon handelt, und dass das Peptid oder das Fragment zellproliferativ auf primäre Osteoblasten aus Rattencalvarien wirkt und/oder zellprotektiv und/oder zelldifferenzierend auf primäre Nervenzellkulturen aus Hinterwurzelganglien von Hühnerembryonen wirkt, und eine der Sequenzen Cadherin-1 human (28-154): Cadherin-2 human (24-159): Cadherin-3 human (27-107): Cadherin-4 human (21-169): Cadherin-5 human (26-47): Cadherin-6 human (19-53): Cadherin-6 human (19-51): Cadherin-8 human: Cadherin-B human (Cadherin-11) Precursor (23-53): Cadherin-C human (Cadherin-12) - Brain-Cadherin Precursor (24-54): Cadherin-C human (Cadherin-12) - Brain-Cadherin Precursor (24-52): Cadherin-D human (Cadherin 13) (23-138): oder Cadherin-F human (Cadherin 14) (22-60): aufweist.

2. Nukleinsäuren codierend für CDGF gemäß Anspruch 1.

3. Nukleinsäure **dadurch gekennzeichnet, dass** sie komplementär zur Nukleinsäure gemäß Anspruch 2 ist.

4. Vektoren enthaltend Nukleinsäuren gemäß mindestens einem der Ansprüche 2 oder 3.

5. Antikörper **dadurch gekennzeichnet, dass** sie gegen CDGF gemäß Anspruch 1 gerichtet sind.

6. Arzneimittel enthaltend CDGF gemäß Anspruch 1, Nukleinsäuren gemäß mindestens einem der Ansprüche 2 oder 3 und/oder Antikörper gemäß Anspruch 5 zusammen mit üblichen Hilfsmitteln.

7. Diagnostikmittel enthaltend CDGF gemäß Anspruch 1, Nukleinsäuren gemäß mindestens einem der Ansprüche 2 oder 3 und/oder Antikörper gemäß Anspruch 5 zusammen mit üblichen Hilfsmitteln.

8. Arzneimittel gemäß Anspruch 6 in geeigneten galenischen Zubereitungen zur oralen, intravenösen, intramuskulären, intracutanen, intrathekalen Anwendung sowie als Aerosol zur transpulmonalen Applikation.

9. Verfahren zur Herstellung von CDGF gemäß Anspruch 1,
- aus Hämofiltrat durch Kationenaustauscher-Extraktion mit nachfolgender Elution der adsorbierten Substanzen, eine erneute Kationenaustauscher-Chromatographie des die Peptide enthaltenden Extraktes sowie mehrstufige Umkehrphasen-Chromatographie oder
- durch Festphasensynthese im Sinne der Merrifield-Synthese sowie Flüssigphasensynthese nach dem Fachmann an sich bekannten Methoden mit geschützten Aminosäuren und Aufreinigung oder
- durch dem Fachmann an sich bekannte Verfahren der heterologen Expression mittels gängiger biotechnologischer Vektoren gemäß Anspruch 5.

## Claims

1. A peptide referred to as cadherin-derived growth factor (CDGF), **characterized by** being a peptide cleaved off as a pro sequence during the processing of pro-cadherin into cadherin, or a fragment thereof, and that said peptide or fragment has a cell-proliferative effect on primary osteoblasts from rat calvarias and/or a cell-protective and/or cell-differentiating effect on primary nerve cell cultures from spinal ganglia of chicken embryos, and has one of the sequences Cadherin-1 human (28-154): Cadherin-2 human (24-159): Cadherin-3 human (27-107): Cadherin-4 human (21-169): Cadherin-5 human (26-47): Cadherin-6 human (19-53): Cadherin-6 human (19-51): Cadherin-8 human: Cadherin-B human (Cadherin-11) Precursor (23-53): Cadherin-C human (Cadherin-12) - Brain-Cadherin Precursor (24-54): Cadherin-C human (Cadherin-12) - Brain-Cadherin Precursor (24-52): Cadherin-D human (Cadherin 13) (23-138): or Cadherin-F human (Cadherin 14) (22-60):

2. Nucleic acids coding for CDGF according to claim 1.

3. A nucleic acid, **characterized by** being complementary to the nucleic acid according to claim 2.

4. Vectors containing nucleic acids according to at least one of claims 2 or 3.

5. Antibodies, **characterized by** being directed against CDGF according to claim 1.

6. A medicament containing CDGF according to claim 1, nucleic acids according to at least one of claims 2 or 3 and/or antibodies according to claim 5 together with usual auxiliary agents.

7. A diagnostic agent containing CDGF according to claim 1, nucleic acids according to at least one of claims 2 or 3 and/or antibodies according to claim 5 together with usual auxiliary agents.

8. The medicament according to claim 6 in suitable galenic formulations for oral, intravenous, intramuscular, intracutaneous, intrathecal administrations, and as an aerosol for transpulmonary administration.

9. A process for the preparation of CDGF according to claim 1
- from hemofiltrate using cation-exchange extraction followed by elution of the adsorbed substances, renewed cation-exchange chromatography of the extract containing the peptides, and multistage reversed-phase chromatography; or
- by solid-phase synthesis in terms of Merrifield synthesis, or liquid-phase synthesis according to methods involving protected amino acids, per se known to those skilled in the art, followed by purification; or
- by methods of heterologous expression, per se known to those skilled in the art, using common biotechnological vectors according to claim 5.

## Revendications

1. Peptide caractérisé comme facteur de croissance dérivé de la cadhérine ("Cadherin Derived Growth factor" :CDGF), **caractérisé en ce qu'**il s'agit d'un peptide qui est divisé en tant que proséquence lors du traitement de la procadhérine en cadhérine ou d'un fragment de celui-ci, et **en ce que** le peptide ou le fragment agit par prolifération cellulaire sur des ostéoblastes primaires issus de calottes crâniennes de rats et/ou agit par protection cellulaire et/ou par différenciation cellulaire sur des cultures de cellules nerveuses primaires issues de ganglions de la racine postérieure d'embryons de poules, et présente l'une des séquences :
cadhérine-1 humaine (28-154) : cadhérine-2 humaine (24-159) : cadhérine-3 humaine (27-107) : cadhérine-4 humaine (21-169) : cadhérine-5 humaine (26-47) : cadhérine-6 humaine (19-53) :
cadhérine-6 humaine (19-51) : cadhérine-8 humaine : précurseur de la cadhérine-B humaine (cadhérine-11) (23-53) : cadhérine-C humaine (cadhérine-12) - précurseur de la cadhérine du cerveau (24-54) : cadhérine-C humaine (cadhérine-12) - précurseur de la cadhérine du cerveau (24-52) : cadhérine-D humaine (cadhérine-13) (23-138) :
ou cadhérine-F humaine (cadhérine-14) (22-60) :

2. Acides nucléiques codant pour le CDGF selon la revendication 1.

3. Acide nucléique **caractérisé en ce qu'**il est complémentaire à l'acide nucléique selon la revendication 2.

4. Vecteurs contenant des acides nucléiques selon au moins l'une des revendications 2 ou 3.

5. Anticorps **caractérisés en ce qu'**ils sont dirigés contre le CDGF selon la revendication 1.

6. Médicament contenant le CDGF selon la revendication 1, des acides nucléiques selon au moins l'une des revendications 2 ou 3 et/ou des anticorps selon la revendication 5 associés à des auxiliaires habituels.

7. Moyen diagnostique contenant le CDGF selon la revendication 1, des acides nucléiques selon au moins l'une des revendications 2 ou 3 et/ou des anticorps selon la revendication 5 associés à des auxiliaires habituels.

8. Médicament selon la revendication 6, dans des préparations galéniques adaptées à une utilisation orale, intraveineuse, intramusculaire, intracutanée, intrathécale ainsi que comme aérosol pour une application transpulmonaire.

9. Procédé pour la préparation de CDGF selon la revendication 1,
- à partir d'un hémofiltrat au moyen d'une extraction sur échangeur de cations avec une élution successive des substances adsorbées, d'une chromatographie sur échangeur de cations répétée de l'extrait contenant les peptides ainsi que d'une chromatographie en phase inverse à plusieurs étapes ou
- au moyen d'une synthèse en phase solide au sens de la méthode de Merrifield ainsi que d'une synthèse en phase liquide selon des méthodes connues en soi par l'homme du métier avec des acides aminés protégés et une purification ou
- au moyen du procédé de l'expression hétérologue connu en soi par l'homme du métier grâce à des vecteurs biotechnologiques habituels selon la revendication 5.


### SEQUÉNZPROTOKOLL

(1) ALLGEMEINE ANGABEN:
   (i) ANMELDER:
      (A) NAME: Wolf-Georg Forssmann
      (B) STRASSE: Feodor-Lynen-Strasse 31
      (C) ORT: Hannover
      (E) LAND: Deutschland
      (F) POSTLEITZAHL: 30625
   (ii) BEZEICHNUNG DER ERFINDUNG: Cadherin derived growth factor und seine Verwendung
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 123 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 1:
   Cys His Pro Gly Phe Asp Ala Glu Ser Tyr Thr Phe Thr Val Pro Arg
    1               5                  10                  15
   Arg His Leu Glu Arg Gly Arg Val Leu Gly Arg Val Asn Phe Cys Thr
               20                  25                  30
   Gly Arg Gln Arg Thr Ala Tyr Phe Ser Leu Asp Thr Arg Phe Lys Val
           35                  40                  45
   Gly Thr Asp Gly Val Ile Thr Val Lys Arg Pro Leu Arg Phe His Asn
       50                  55                  60
   Pro Gln Ile His Phe Leu Val Tyr Ala Trp Asp Ser Thr Tyr Arg Lys
   65                  70                  75                  80
   Phe Ser Thr Lys Val Thr Leu Asn Gly His His His Arg Pro Pro Pro
                   85                  90                  95
   His Gln Ala Ser Val Ser Gly Ile Gln Ala Glu Leu Leu Thr Phe Pro
               100                 105                 110
   Asn Ser Ser Pro Gly Leu Arg Arg Gln Lys Arg
           115                 120
(2) ANGABEN ZU SEQ ID NO: 2:
   (i) SEQUENZKENNZEICHEN:
      (A) LANGE: 132 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 2:
   Glu Ala Ser Gly Glu Ile Ala Leu Cys Lys Thr Gly Phe Pro Glu Asp
   1               5                   10                  15
   Val Tyr Ser Ala Val Leu Ser Lys Asp Val His Glu Gly Gln Pro Leu
               20                  25                  30
   Leu Asn Val Phe Ser Asn Cys Asn Gly Lys Arg Lys Val Gln Tyr Glu
           35                  40                  45
   Ser Ser Glu Pro Ala Asp Phe Lys Val Asp Glu Asp Gly Met Val Tyr
       50                  55                  60
   Ala Val Arg Ser Phe Pro Leu Ser Ser Glu His Ala Lys Phe Leu Ile
   65                  70                  75                  80
   Tyr Ala Gln Asp Lys Glu Thr Gln Glu Lys Trp Gln Lys Leu Ser Leu
                   85                  90                  95
   Lys Pro Thr Leu Thr Glu Glu Ser Val Lys Glu Ser Ala Glu Val Glu
               100                 105                 110
   Glu Ile Val Phe Pro Arg Gln Phe Ser Lys His Ser Gly His Leu Gln
           115                 120                 125
   Arg Gln Lys Arg
       130
(2) ANGABEN ZU SEQ ID NO: 3:
   (i) SEQUENZKENNZEICHEN:
      (A) LÄNGE: 78 Aminosäuren
      (B) ART: Aminosäure
      (C) STRANGFORM: nicht bekannt
      (D) TOPOLOGIE: nicht bekannt
   (ii) ART DES MOLEK 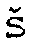 LS: Peptid
   (xi) SEQUENZBESCHREIBUNG: SEQ ID NO: 3:
   Cys Arg Ala Val Phe Arg Glu Ala Glu Val Thr Leu Glu Ala Gly Gly
     1               5                  10                  15
   Ala Glu Gln Glu Pro Gly Gln Ala Leu Gly Lys Val Phe Met Gly Gln
               20                  25                  30
   Glu Pro Ala Leu Phe Ser Thr Asp Asn Asp Asp Phe Thr Val Arg Asn
           35                  40                  45
   Gly Glu Thr Val Gln Glu Arg Arg Ser Leu Lys Glu Arg Asn Pro Leu
       50                  55                  60
   Lys Ile Phe Pro Ser Lys Arg Ile Leu Arg Arg His Lys Arg
   65                  70                  75
(2) ANGABEN ZU SEQ ID NO: 4:
   (i) SEQUENZKENNZEICHEN:
      (A) LÄNGE: 144 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 4:
   His Asn Glu Asp Leu Thr Thr Arg Glu Thr Cys Lys Ala Gly Phe Ser
     1               5                  10                  15
   Glu Asp Asp Tyr Thr Ala Leu Ile Ser Gln Asn Ile Leu Glu Gly Glu
   20 25 30
   Lys Leu Leu Gln Val Lys Ser Ser Cys Val Gly Thr Lys Gly Thr Gln
   35 40 45
   Tyr Glu Thr Asn Ser Met Asp Phe Lys Gly Ala Asp Gly Thr Val Phe
   50 55 60
   Ala Thr Arg Glu Leu Gln Val Pro Ser Glu Gln Val Ala Phe Thr Val
   65 70 75 80
   Thr Ala Trp Asp Ser Gln Thr Ala Glu Lys Trp Asp Ala Val Leu Val
   85 90 95
   Ala Gln Thr Ser Ser Pro His Ser Gly His Lys Pro Gln Lys Gly Lys
   100 105 110
   Lys Val Val Ala Leu Asp Pro Ser Pro Pro Pro Lys Asp Thr Leu Leu
   115 120 125
   Pro Trp Pro Gln His Gln Asn Ala Asn Gly Leu Arg Arg Arg Lys Arg
   130 135 140
(2) ANGABEN ZU SEQ ID NO: 5:
   (i) SEQUENZKENNZEICHEN:
      (A) LÄNGE: 22 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 5:
   Ala Gly Ala Asn Pro Ala Gln Arg Asp Thr His Ser Leu Leu Pro Thr
   1 5 10 15
   His Arg Arg Gln Lys Arg
   20
(2) ANGABEN ZU SEQ ID NO: 6:
   (i) SEQUENZKENNZEICHEN:
      (A) LÄNGE: 35 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 6:
   Thr Leu Ser Thr Pro Leu Ser Lys Arg Thr Ser Gly Phe Pro Ala Lys
   1               5                   10                  15
   Lys Arg Ala Leu Glu Leu Ser Gly Asn Ser Lys Asn Glu Leu Asn Arg
               20                  25                  30
   Ser Lys Arg
           35
(2) ANGABEN ZU SEQ ID NO: 7:
   (i) SEQUENZKENNZEICHEN:
      (A) LÄNGE: 33 Aminosäuren
      (B) ART: Aminosäure
      (C) STRANGFORM: nicht bekannt
      (D) TOPOLOGIE: nicht bekannt

   (ii) ART DES MOLEKÜLS: Peptid
   (xi) SEQUENZBESCHREIBUNG: SEQ ID NO: 7:
   Thr Leu Ser Thr Pro Leu Ser Lys Arg Thr Ser Gly Phe Pro Ala Lys
   1               5                   10                  15
   Lys Arg Ala Leu Glu Leu Ser Gly Asn Ser Lys Asn Glu Leu Asn Arg
               20                  25                  30
   Ser
(2) ANGABEN ZU SEQ ID NO: 8:
   (i) SEQUENZKENNZEICHEN:
      (A) LÄNGE: 54 Aminosäuren
      (B) ART: Aminosäure
      (C) STRANGFORM: nicht bekannt
      (D) TOPOLOGIE: nicht bekannt

   (ii) ART DES MOLEKÜLS: Peptid
   (xi) SEQUENZBESCHREIBUNG: SEQ ID NO: 8:
   Met Leu Leu Asp Leu Trp Thr Pro Leu Ile Ile Leu Trp Ile Thr Leu
   1               5                   10                  15
   Pro Pro Cys Ile Tyr Met Ala Pro Met Asn Gln Ser Gln Val Leu Met
               20                  25                  30
   Ser Gly Ser Pro Leu Glu Leu Asn Ser Leu Gly Glu Glu Gln Arg Ile
           35                  40                  45
   Leu Asn Arg Ser Lys Arg
       50
(2) ANGABEN ZU SEQ ID NO: 9:
   (i) SEQUENZKENNZEICHEN:
      (A) LÄNGE: 31 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 9:
   Phe Ala Pro Glu Arg Arg Gly His Leu Arg Pro Ser Phe His Gly His
   1               5                   10                  15
   His Glu Lys Gly Lys Glu Gly Gln Val Leu Gln Arg Ser Lys Arg
               20                  25                  30
(2) ANGABEN ZU SEQ ID NO: 10:
   (i) SEQUENZKENNZEICHEN:
      (A) LÄNGE: 26 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 10:
   Glu Arg Arg Gly His Leu Arg Pro Ser Phe His Gly His His Glu Lys
   1               5                   10                  15
   Gly Lys Glu Gly Gln Val Leu Gln Arg Ser
               20                  25
(2) ANGABEN ZU SEQ ID NO: 11:
   (i) SEQUENZKENNZEICHEN:
      (A) LÄNGE: 31 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 11:
   Gln Pro Gln Pro Gln Gln Thr Leu Ala Thr Glu Pro Arg Glu Asn Val
   1               5                   10                  15
   Ile His Leu Pro Gly Gln Arg Ser His Phe Gln Arg Val Lys Arg
               20                  25                  30
(2) ANGABEN ZU SEQ ID NO: 12:
   (i) SEQUENZKENNZEICHEN:
      (A) LÄNGE: 29 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 12:
   Gln Pro Gln Pro Gln Gln Thr Leu Ala Thr Glu Pro Arg Glu Asn Val
   1               5                   10                  15
   Ile His Leu Pro Gly Gln Arg Ser His Phe Gln Arg Val
               20                  25
(2) ANGABEN ZU SEQ ID NO: 13:
   (i) SEQUENZKENNZEICHEN:
      (A) LÄNGE: 129 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 13:
   Glu Asp Leu Asp Cys Thr Pro Gly Phe Gln Gln Lys Val Phe His Ile
   1               5                   10                  15
   Asn Gln Pro Ala Glu Phe Ile Glu Asp Gln Ser Ile Leu Asn Leu Thr
               20                  25                  30
   Phe Ser Asp Cys Lys Gly Asn Asp Lys Leu Arg Tyr Glu Val Ser Ser
           35                  40                  45
   Pro Tyr Phe Lys Val Asn Ser Asp Gly Gly Leu Val Ala Leu Arg Asn
       50                  55                  60
   Ile Thr Ala Val Gly Lys Thr Leu Phe Val His Ala Arg Thr Pro His
   65                  70                  75                  80
   Ala Glu Phe Asp Met Ala Glu Leu Val Ile Val Gly Gly Lys Asp Ile
                   85                  90                  95
   Ser Leu Gln Asp Ile Phe Lys Phe Ala Arg Thr Ser Pro Val Pro Arg
               100                 105                 110
   Gln Lys Arg Pro Ser Val Leu Leu Leu Ser Leu Phe Ser Leu Ala Cys
           115                 120                 125
   Leu
(2) ANGABEN ZU SEQ ID NO: 14:
   (i) SEQUENZKENNZEICHEN:
      (A) LÄNGE: 39 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (xi) SEQUENZBESCHREIBUNG: SEQ ID NO: 14:
   Val Pro Gly Trp Arg Arg Pro Thr Thr Leu Tyr Pro Trp Arg Arg Ala
   1               5                   10                  15
   Pro Ala Leu Ser Arg Val Arg Arg Ala Trp Val Ile Pro Pro Ile Ser
               20                  25                  30
   Val Ser Glu Asn His Lys Arg
           35